(19) Europäisches Patentamt
European Patent Office
Office européen des brevets

(11) Veröffentlichungsnummer: **0 017 806**
**B1**

(12) # EUROPÄISCHE PATENTSCHRIFT

(45) Veröffentlichungstag der Patentschrift: **02.09.81**

(51) Int. Cl.³: **C 07 C 97/24**

(21) Anmeldenummer: **80101559.5**

(22) Anmeldetag: **25.03.80**

(54) **Verfahren zur Herstellung von 1-Amino-2-acetyl-4-bromanthrachinon.**

(30) Priorität: **29.03.79 DE 2912570**

(43) Veröffentlichungstag der Anmeldung: **29.10.80 Patentblatt 80/22**

(45) Bekanntmachung des Hinweises auf die Patenterteilung: **02.09.81 Patentblatt 81/35**

(84) Benannte Vertragsstaaten: **CH DE GB**

(56) Entgegenhaltungen:
**DE-A-1 668 870**
**DE-C-612 870**
**DE-C-1 127 911**
**FR-A-839 943**
**US-A-1 830 152**
**US-A-1 830 153**

(73) Patentinhaber: **BASF Aktiengesellschaft, Carl-Bosch-Strasse 38, D-6700 Ludwigshafen (DE)**

(72) Erfinder: **Epple, Gerhard, Dr., Karl-Gayer-Strasse 8, D-6719 Weisenheim (DE)**
Erfinder: **Flohr, Helmut, Dr., Gellertstrasse 46, D-7500 Karlsruhe (DE)**

**EP 0 017 806 B1**

Anmerkung: Innerhalb von neun Monaten nach der Bekanntmachung des Hinweises auf die Erteilung des europäischer Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von 1-Amino-2-acetyl-4-brom-anthrachinon

Die Erfindung betrifft ein Verfahren zur Herstellung von 1-Amino-2-acetyl-4-brom-anthrachinon aus 3-Methyl-anthra-[1,2-c]-isoxazol.

1-Amino-2-acetyl-4-brom-anthrachinon ist ein Zwischenprodukt für die Herstellung von Farbstoffen. Durch Umsetzung mit Toluolsulfonamid und anschließender Verseifung entsteht 1,4-Diamino-2-acetyl-anthrachinon, welches als Farbstoff für das Transferverfahren zum Färben von Polyester verwendet wird. Außerdem ist 1,4-Diamino-2-acetylanthrachinon ein Ausgangsprodukt für die Herstellung von wertvollen Küpenfarbstoffen. 1-Amino-2-acetyl-4-brom-anthrachinon wird auf folgendem Wege hergestellt:

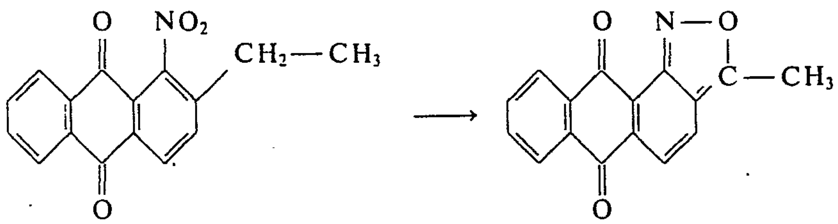

(II) (III)

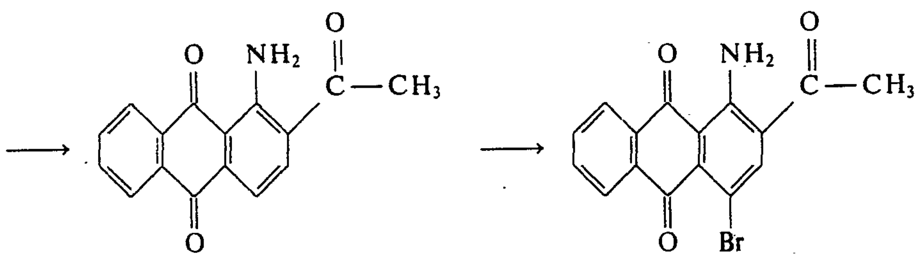

(IV) (I)

Die Überführung von 1-Nitro-2-äthylanthrachinon (II) in 2-Methyl-anthra-[1,2-c]-isoxazol (III) wird in bekannter Weise durchgeführt (US-PS 1 830 152, Beispiel 2).

Die Reduktion der Isoxazolverbindung (III) erfolgt nach den Angaben in den US-PS 1 830 152 und 1 830 153 in wäßriger ammoniakalischer Dithionitlösung. Nach der US-PS 1 830 152, Beispiel 4 kann die aus 1-Nitro-2-methylanthrachinon erhältliche Isoxazolverbindung auch mit einer wäßrigen Eisen-II-sulfatlösung bei 30 bis 40° C reduziert werden.

Verbindung (I) wird in der FR-PS 839 943 und der DE-PS 612 870 als Zwischenprodukt erwähnt, jedoch sind Angaben für eine Herstellung nicht vorhanden.

Aus der DE-PS 1 127 911 ist bekannt, daß (IV) in 70 bis 98gew.-%iger Schwefelsäure oder in Eisessig mit Brom 1-Amino-2-(bromacetyl)-anthrachinon liefert.

Aufgabe der vorliegenden Erfindung war es, ein Verfahren zur Herstellung von 1-Amino-2-acetyl-4-bromanthrachinon bereitzustellen, nach dem das Verfahrensprodukt in hoher Ausbeute und hoher Reinheit und ohne Isolierung von Zwischenstufen erhalten werden kann.

Es wurde gefunden, daß man 1-Amino-2-acetyl-4-bromanthrachinon aus 1-Nitro-2-äthylanthrachinon durch Umlagerung von 1-Nitro-2-äthylanthrachinon in Oleum zu 3-Methyl-anthra-[1,2-c]-isoxazol, Reduktion der Isoxazolverbindung und Bromierung in guter Ausbeute und hoher Reinheit erhält, wenn man in die bei der Herstellung der Isoxazolverbindung entstehende Oleum enthaltende Lösung der Isoxazolverbindung bei Temperaturen zwischen 10 und 30°C die 2- bis 2,5fache Gewichtsmenge, bezogen auf 1-Nitro-2-äthylanthrachinon, kristallisiertes Eisen-II-sulfat einträgt, das Gemisch durch Zugeben von Wasser zu 60 bis 90gew.-%iger Schwefelsäure verdünnt, die Isoxazolverbindung durch Erwärmen auf Temperaturen zwischen 80 und 95°C reduziert und das Reduktionsprodukt in der schwefelsauren Lösung nach dem Verdünnen auf 10 bis 50gew.-%ige Schwefelsäure bei Temperaturen zwischen 5 und 50° C monobromiert.

Das Verfahren liefert (I) in einer Ausbeute von etwa 90 bis zu 98% der Theorie, bezogen auf 1-Nitro-2-äthylanthrachinon, bei gleichzeitig hoher Reinheit. Demgegenüber erhält man 1-Amino-2-acetyl-4-bromanthrachinon über 1-Nitro-2-äthylanthrachinon nach dem in der US-PS 1 830 152 beschriebenen Verfahren zur Herstellung von 1-Amino-2-acetylanthrachinon und dessen Bromierung in 20gew.-%iger Schwefelsäure in ca. 80%iger Ausbeute, wobei das Verfahrensprodukt weniger rein ist als das nach dem erfindungsgemäßen Verfahren erhaltene.

Das erfindungsgemäße Verfahren hat noch einen weiteren Vorteil: man erhält auch aus weniger reinem 1-Nitro-2-äthylanthrachinon, z. B. mit Nitrierprodukten, die noch 3-Nitro-2-äthylanthrachinon

und Ausgangsprodukt enthalten, ein hochwertiges 1-Amino-2-acetyl-4-bromanthrachinon in hoher Ausbeute.

Das Verfahren wird in der Regel in folgender Weise durchgeführt: 1-Nitro-2-äthylanthrachinon (II) wird bei Temperaturen zwischen 0 und 25°C in Oleum eingetragen, worin die Kondensation zur Isoxazolverbindung (III) erfolgt. Nach Beendigung der Reaktion, dies ist im allgemeinen nach 0,5 bis 5 Stunden der Fall, trägt man in die Oleum enthaltende Lösung kristallisiertes Eisen-II-sulfat ein. Die Temperatur soll dabei zwischen 10 und 30°C liegen. Vorteilhafterweise wird die Temperatur während der Zugabe zwischen 15 und 20°C gehalten.

Die Menge an kristallisiertem Eisen-II-sulfat ($FeSO_4 \cdot 7\,H_2O$) beträgt das 2- bis 2,5fache des verwendeten 1-Nitro-2-äthylanthrachinons. Mehr als die 2,5fache Gewichtsmenge an Eisen-II-sulfat erbringen jedoch keinen Vorteil.

Danach gibt man dem Gemisch langsam so viel Wasser zu, daß eine 60 bis 90, vorzugsweise eine 70 bis 90gew.-%ige Schwefelsäure vorliegt. Das Gemisch wird dann auf Temperaturen zwischen 60 und 95°C erwärmt und in diesem Temperaturbereich gehalten bis die Reduktion beendet ist.

Die Reduktion erfolgt vorzugsweise bei Temperaturen zwischen 80 und 95, insbesondere zwischen 80 und 90°C. Bei diesen Temperaturen ist die Reduktion im allgemeinen nach 1 bis 4 Stunden beendet.

Nach Beendigung der Reduktion wird das Reduktionsgemisch mit Wasser verdünnt, daß eine Suspension in 10 bis 50, vorzugsweise 25 bis 40gew.-%iger Schwefelsäure vorliegt. Das Verdünnen kann durch Zugeben von Wasser zum Reduktionsgemisch oder durch Eintragen des Reduktionsgemisches in die entsprechende Menge Wasser erfolgen. Da die Bromierung bei Temperaturen unterhalb 50, vorzugsweise bei 30 bis 15°C erfolgen soll, verwendet man zweckmäßigerweise kaltes Wasser, Eiswasser oder ein Gemisch aus Eis und Wasser zum Verdünnen.

Die Bromierung erfolgt mit Brom oder mit einer Lösung von Alkalimetallbromid und Natriumbromat im Verhältnis 2 : 1 Mol. Die Bromierung erfolgt selektiv am Anthrachinonkern.

Die Bromierung erfolgt vorteilhafterweise bei Temperaturen zwischen 30 und 15°C, insbesondere bei Raumtemperatur (20 bis 25°C), wobei das Brom, bzw. die Bromid-Bromat-Lösung im Verlauf von 1 bis 5 Stunden kontinuierlich oder in Portionen zugegeben wird.

Die Menge an Brom bzw. an Bromid-Bromat beträgt mindestens 1 Mol je Mol Anthrachinonverbindung. Vorteilhafterweise wendet man 1,0 bis 1,3 Mol Brom bzw. die dazu äquivalente Menge an Bromid-Bromat an.

Nach der Zugabe des Bromierungsmittels wird 2 bis 5 Stunden nachgerührt, ein vorhandener Überschuß an Brom wird zweckmäßigerweise vor der Isolierung durch Zugeben von Alkalimetallhydrogensulfit entfernt und dann das Verfahrensprodukt in üblicher Weise isoliert, z. B. durch Filtrieren. Der Filterkuchen wird mit heißem Wasser neutral gewaschen. Ein Überschuß an Brom kann auch durch Waschen des Filtergutes mit Natriumhydrogensulfitlösung entfernt werden.

Das nach dem erfindungsgemäßen Verfahren erhaltene 1-Amino-2-acetyl-4-bromanthrachinon läßt sich mit Toluolsulfonsäureamid in die entsprechende Sulfonamidoverbindung und diese durch Verseifen in Schwefelsäure in 1,4-Diamino-2-acetylanthrachinon von hoher Reinheit überführen. Nach dem Verfahren gemäß der vorliegenden Erfindung kann 1,4-Diamino-2-acetylanthrachinon ausgehend von 1-Nitro-2-äthylanthrachinon in hoher Ausbeute und sehr guter Reinheit mit verhältnismäßig geringem technischem Aufwand hergestellt werden. Ein entsprechend gutes Ergebnis wird erzielt, wenn man als Ausgangsprodukt weniger reines 1-Nitro-2-äthylanthrachinon verwendet.

Das Verfahren gemäß der Erfindung wird durch die folgenden Beispiele weiter erläutert. Die Prozentangaben beziehen sich auf das Gewicht.

## Beispiel 1

In eine Mischung aus 210 g 65%iges Oleum und 137 g 96%iger Schwefelsäure werden unter Rühren und Solekühlung bei 15 bis 20°C 45 g 1-Nitro-2-äthyl-anthrachinon innerhalb von 1/2 Stunde eingetragen. Man rührt 2 Stunden bei dieser Temperatur nach und trägt bei 15 bis 20°C 89 g Eisenvitriol ($FeSO_4 \cdot 7\,H_2O$) ein. Anschließend tropft man 93 g Wasser zu und erhitzt auf 80 bis 85°C und hält bei 85°C bis zur vollständigen Reduktion (2 Stunden). Das Reaktionsgemisch wird auf 1 kg Eiswasser gegossen. Zu der Suspension wird im Verlauf von 2 Stunden bei Raumtemperatur eine Lösung von 7,89 g Natriumbromat und 11,56 g Natriumbromid in 50 g Wasser gegeben und das Reaktionsgemisch 3 Stunden nachgerührt. Das ausgefallene Reaktionsprodukt wird abgesaugt, mit 30 g 40%iger Natriumhydrogensulfitlösung, anschließend mit heißem Wasser neutral gewaschen und getrocknet. Ausbeute: 54 g 1-Amino-2-acetyl-4-brom-anthrachinon (entsprechend 98% der Theorie, bezogen auf 1-Nitro-2-äthylanthrachinon); Bromgehalt 20,2% (ber. 23,2%).

Durch Umsetzung dieses Produkts mit Toluolsulfonsäureamid in o-Dichlorbenzol erhält man in 75,6%iger Ausbeute 1-Amino-2-acetyl-4-toluolsolfonamido-anthrachinon, das nach Verseifung in Schwefelsäure in 92,9%iger Ausbeute ein 98%iges 1,4-Diamino-2-acetyl-anthrachinon liefert.

Beispiel 2

Man arbeitet wie in Beispiel 1 angegeben, bromiert jedoch mit 25,6 g Brom. Ausbeute: 51 g 1-Amino-2-acetyl-4-brom-anthrachinon (entsprechend 92,6% der Theorie), Bromgehalt: 24,5% (ber. 23,2%).

Beispiel 3

a) In eine Mischung von 411 g 65%igem Oleum und 268 g 95%iger Schwefelsäure werden unter Rühren und Kühlen 88 g 80,2%iges 1-Nitro-2-äthylanthrachinon eingetragen. Man rührt 2 Stunden bei dieser Temperatur nach und trägt bei 20 bis 25° C 174 g Eisenvitriol ein. Anschließend tropft man 181 g Wasser zu und erhitzt 2 Stunden auf 80 bis 85° C. Das Reaktionsgemisch wird auf 2 l Eiswasser gegossen und innerhalb von 2 Stunden mit einer Lösung von 16,23 g Natriumbromat und 23,7 g Natriumbromid in 195 ml Wasser versetzt. Man rührt 1 Stunde nach, saugt das ausgefallene Reaktionsprodukt ab und wäscht mit heißem Wasser neutral. Nach dem Trocknen erhält man 92 g 1-Amino-2-acetyl-4-brom-anthrachinon.
Aus diesem Produkt erhält man in 60,1%iger Ausbeute 1-Amino-2-acetyl-4-tosylamido-anthrachinon, das nach der Verseifung in Schwefelsäure in 97,1%iger Ausbeute ein 94,6%iges 1,4-Diamino-2-acetyl-anthrachinon liefert.

b) 1-Amino-2-acetyl-4-bromanthrachinon
26,5 g 1-Amino-2-acetylanthrachinon werden in 285 g 20%iger Schwefelsäure suspendiert. Unter Rühren werden bei Raumtemperatur im Verlauf von 2 Stunden 16,0 g Brom zugetropft. Nach 2stündigem Rühren gibt man weitere 1,6 g Brom zu und rührt 2 Stunden nach. Das Verfahrensprodukt wird dann abgesaugt, mit heißem Wasser neutral gewaschen und getrocknet.
Ausbeute: 32,7 g 1-Amino-2-acetyl-4-bromanthrachinon (entsprechend 95,1% der Theorie) mit einem Bromgehalt von 21,4% (ber. 23,2%).
Aus dem Bromderivat erhält man duzrch Umsetzen mit o-/p-Toluolsulfonsäureamid die entsprechende Sulfonamidoverbindung, die durch Verseifung in 96%iger Schwefelsäure 1,4-Diamino-2-acetylanthrachinon in 52%iger Ausbeute mit einem Reingehalt von 89,3% liefert (bezogen auf 1-Amino-2-acetyl-4-bromanthrachinon).

**Patentansprüche**

1. Verfahren zur Herstellung von 1-Amino-2-acetyl-4-bromanthrachinon durch Umsetzung von 1-Nitro-2-äthylanthrachinon in Oleum zu 3-Methyl-anthra-[1,2-c]-isoxazol, Reduktion des Isoxazolderivates mit Eisen-2-sulfat und Bromierung des 1-Amino-2-acetyl-anthrachinons, dadurch gekennzeichnet, daß man in die Oleum enthaltende Lösung der Isoxazolverbindung bei Temperaturen zwischen 10 und 30° C die 2- bis 2,5fache Gewichtsmenge, bezogen auf 1-Nitro-2-äthylanthrachinon, kristallisiertes Eisen-II-sulfat einträgt, das Gemisch durch Zugeben von Wasser zu 60 bis 90gew.-%iger Schwefelsäure verdünnt, die Isoxazolverbindung durch Erwärmen auf Temperaturen zwischen 80 und 95° C reduziert und das Reaktionsprodukt in der schwefelsauren Lösung nach dem Verdünnen auf 10 bis 50gew.-%ige Schwefelsäure bei Temperaturen zwischen 5 und 50° C monobromiert.

2. Verfahren nach Anspruch 1, dadurch gekennzeichnet, daß die Reduktion in 70 bis 90gew.-%iger Schwefelsäure erfolgt.

3. Verfahren nach Anspruch 1 oder 2, dadurch gekennzeichnet, daß die Reduktion bei Temperaturen zwischen 80 und 90° C erfolgt.

4. Verfahren nach Anspruch 1, 2 oder 3, dadurch gekennzeichnet, daß die Bromierung in 25 bis 40gew.-%iger Schwefelsäure bei Temperaturen zwischen 30 und 15° C erfolgt.

**Claims**

1. A process for the production of 1-Amino-2-acetyl-4-bromoanthraquinone by reacting 1-nitro-2-ethylanthraquinone in oleum to form 3-methylanthra-[1,2-c]-isoxazole, reducing the isoxazole derivative with iron(II) sulphate and brominating the 1-amino-2-acetylanthraquinone, characterized in that 2 to 2¹/₂ times the weight of 1-nitro-2-ethylanthraquinone of crystallized iron(II) sulphate is introduced into the oleum-containing solution of the isoxazole compound at temperatures of from 10° to 30° C, the mixture ist diluted with water to give 60 to 90% by weight strenght sulphuric acid, the isoxazole compound is reduced by heating at temperatures of from 80° to 95° C, and the reduction product is monobrominated at temperatures of from 5° to 50° C in the sulphuric acid solution after dilution to 10 to 50% by weight strength sulphuric acid.

2. A process as claimed in claim 1, characterized in that the reduction is carried out in 70 to 90% weight strength sulphuric acid.

3. A process as claimed in claim 1 or 2, characterized in that the reduction is carried out at temperatures of from 80° to 90° C.

4. A process as claimed in claim 1, 2 or 3, characterized in that the bromination is carried out in 25 to 40% by weight strength sulphuric acid at temperatures of from 15° to 30° C.

**Revendications**

1. Procédé pour la préparation de la 1-amino-2-acétyl-4-bromoanthraquinone par conversion de la 1-nitro-2-éthylanthraquinone dans l'oléum en 3-methylanthra-[1,2-c]-isoxazole, réduction du dérivé d'isoxazole à l'aidè du sulfate ferreux et bromation de la 1-amino-2-acétyl-anthraquinone, caractérisé en ce que l'on introduit le sulfate ferreux cristallisé dans la solution, contenant de l'oléum, du composé d'isoxazole à des températures de 10 à 30° C et en quantité de 2 à 2,5 fois le poids de la 1-nitro-2-éthylanthraquinone; on dilue le mélange par addition d'eau à une concentration de 60 à 90% en poids en acide sulfurique, on réduit le composé d'isoxazole par chauffage à des températures de 80 à 95° C et on soumet le produit de réduction à monobromation à des températures de 5 à 50° C dans la solution sulfurique aprés dilution à une concentration de 10 à 50% en poids d'acide sulfurique.

2. Procédé selon la revendication 1, caractérisé en ce que l'on effectue la réduction dans l'acide sulfurique à une concentration de 70 à 90% en poids.

3. Procédé selon la revendication 1 ou 2, caractérisé en ce que l'on effectue la réduction à des températures de 80 à 90° C.

4. Procédé selon la revendication 1, 2 ou 3, caractérisé en ce que l'on effectue la bromation dans l'acide sulfurique à une concentration de 25 à 40% en poids à des températures de 30 à 15° C.